(19) Europäisches Patentamt
European Patent Office
Office européen des brevets

(11) Veröffentlichungsnummer: **0 347 524 B1**

(12) **EUROPÄISCHE PATENTSCHRIFT**

(45) Veröffentlichungstag der Patentschrift: **28.06.95**

(51) Int. Cl.[6]: **C07D 251/38**, C08K 5/37

(21) Anmeldenummer: **89104878.7**

(22) Anmeldetag: **18.03.89**

# (54) Bis(2,4-organylthio-s-triazin-6-yl)polysulfane, Verfahren zu ihrer Herstellung und sie enthaltende vulkanisierbare Kautschukmischungen.

(30) Priorität: **22.06.88 DE 3820969**

(43) Veröffentlichungstag der Anmeldung:
**27.12.89 Patentblatt 89/52**

(45) Bekanntmachung des Hinweises auf die Patenterteilung:
**28.06.95 Patentblatt 95/26**

(84) Benannte Vertragsstaaten:
**AT DE ES FR GB**

(56) Entgegenhaltungen:
**US-A- 4 621 121**

**CHEMICAL ABSTRACTS, Band 68, 1968, Seite 10155, Spalte 2, Zusammenfassung Nr.105172q, Columbus, Ohio, US; A.P. ANTYKOV: "Structure and some properties ofpseudothiocyanogen", & ZH. PRIKL. KHIM. 40(11), 2547-**

**CHEMICAL ABSTRACTS, Band 87, 1977, Seite 42, Spalte 2, Zusammenfassung Nr.54144q, Columbus, Ohio, US; &JP-A-77 49 260 (ADEKA ARGUS CHEMICAL CO.) 20-04-1977**

(73) Patentinhaber: **Degussa Aktiengesellschaft**
**Weissfrauenstrasse 9**
**D-60311 Frankturt (DE)**

(72) Erfinder: **Hellwig, Georg, Dr.**
**Wilh.-Busch-Strasse 3**
**D-6463 Freigericht 1 (DE)**
Erfinder: **Stober, Reinhard, Dr.**
**Bornwiesenweg 22**
**D-6467 Hasselroth 2 (DE)**
Erfinder: **Klatte, Christoph**
**August-Bebel-Strasse 2a**
**D-6450 Hanau 1 (DE)**
Erfinder: **Deschler, Ulrich, Dr.**
**Birkenweg 1**
**D-6450 Hanau 9 (DE)**
Erfinder: **Wolff, Siegfried, Dr.**
**Weiherstrasse 28**
**D-5303 Bornheim (DE)**
Erfinder: **Görl, Udo, Dr.**
**Ouittenstrasse 36**
**D-5309 Meckenheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 347 524 B1

CHEMICAL ABSTRACTS, Band 100, 1984, Seite 76, Spalte 1, Zusammenfassung Nr.8250k, Columbus, Ohio, US; &JP-A-58 93 739 (SANCHIN CHEMICAL INDUSTRY CO.) 03-06-1983

CHEMICAL ABSTRACTS, Band 98, 1983, Seite 517, Spalte 1, Zusammenfassung Nr.160742v, Columbus, Ohio, US; &JP-A-57 175 174 (JAPAN SYNTHETIC RUBBER CO.) 28-10-1982

## Beschreibung

Die Erfindung betrifft zweikernige s-Triazinverbindungen, die über eine Sulfankette verknüpft sind, ein Verfahren zur Herstellung und sie enthaltende vulkanisierbare Kautschukmischungen.

Zweikernige s-Triazinverbindungen mit einer Sulfankette sind zum Beispiel aus der DE-PS 1 669 954, US-PS 3,923,724 und der US PS 4,621,121 bekannt.
Es handelt sich dabei um das Bis(2-ethylamino-4-diethylamino-s-triazin-6-yl)disulfan beziehungsweise das entsprechende Tetrasulfan, die in vulkanisierbaren Kautschukmischungen als Vulkanisationsbeschleuniger eingesetzt werden.

Aus Zh. Prikl. Khim 40 (11)2547-2552 (1967) (zit. nach C.A., Band 68, 1968, Seite 10155, Nr. 105172q) sind die Verbindungen Bis(2,4-dimethylthio-s-triazin-6-yl)disulfid und Bis(2,4-dithiol-s-triazin-6-yl)disulfid bekannt.
Eine Verwendung als Vulkanisationshilfsmittel wird nicht beschrieben.

Aufgabe der Erfindung sind Vulkanisationsbeschleuniger die aufgrund höherer Scorchzeiten und Anvulkanisationszeiten die Verarbeitung von Kautschukmischungen erleichtern.

Gegenstand der Erfindung sind Bis(2,4-organylthio-s-triazin-6-yl)polysulfane der allgemeinen Formel (I)

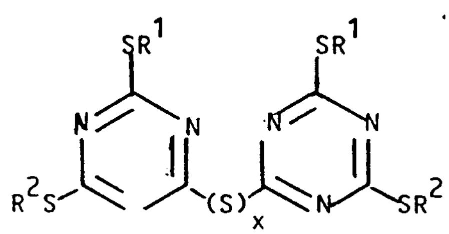

in der bedeuten:
$R^1$, $R^2$: gleich oder verschieden, H, Alkyl mit 1-4 C-Atomen, verzweigt oder unverzweigt, $C_3$-$C_8$ Cycloalkyl,insbesondere Cyclohexyl, Phenyl oder auch 2-Hydroxyethyl, 3-Hydroxypropyl, 2-Hydroxypropyl, $(S)_x$ eine Polysulfankette mit 2 - 10 C-Atomen ($2 \leqq x \leqq 10$), wobei die einzelnen Polysulfane in solchen Konzentrationen vorliegen, daß das statistische Mittel $\bar{x}$ ganze oder gebrochene Zahlenwerte von 2 bis 5 annimmt, insbesondere aber ~ 4 entspricht, mit Ausnahme des Bis(2,4-dimethylthio-s-triazin-6-yl)disulfids und des Bis(2,4-dithiol-s-triazin-6-yl)disulfides. Ein weiterer Gegenstand der Erfindung ist das Verfahren zur Herstellung von Verbindungen gemäß Anspruch 1, das dadurch gekennzeichnet ist, daß man eine s-Triazinverbindung der allgemeinen Formel (II)

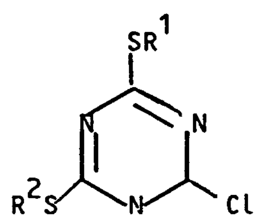

in der $R^1$ und $R^2$ die oben angegebenen Bedeutungen besitzen, in einem organischen Lösungsmittel, bevorzugt einem polaren Lösungsmittel,oder deren Gemischen mit Wasser mit einer polysulfidischen Verbindung der allgemeinen Formel (III)

$Me_2S_x$ ,

in der Me für das Ammonium- oder ein Alkalikation, insbesondere Natrium oder Kalium, steht, und $S_x$ der obengenannten Bedeutung enspricht, insbesondere aber $\bar{x}$ ~ 4 bedeutet, in einem Molverhältnis von 2 : 1 bis 2 : 1,2 bei einer Temperatur von 0 °C bis zum Siedepunkt des organischen Lösungsmittels umsetzt und das Produkt abtrennt und trocknet.

Man geht im allgemeinen so vor, daß man die Ausgangsverbindungen gemäß Formel (II) in dem organischen Lösungsmittel, darunter bevorzugt THF, 1,2 Dimethoxyethan oder Ketone, wie z. B. Aceton,

Methylethylketon und Isobutylmethylketon gelöst vorlegt, anschließend unter kräftigem Rühren das Polysulfid entsprechend Formel (III), bevorzugt Dinatriumtetrasulfid, entweder in fein gepulverter Form oder in Wasser gelöst zusetzt, und die Reaktion bevorzugt bei Temperaturen von 20 bis 50 °C ablaufen läßt. Der Wasseranteil des Reaktionsgemisches sollte 2 bis 40 Gew.-% nicht überschreiten.

Von Bedeutung für die Auswahl des organischen Lösungsmittels ist auch, daß das gewünschte Produkt als Niederschlag anfällt, so daß es ohne Probleme zum Beispiel abfiltriert werden kann.

Die als Ausgangsverbindungen eingesetzten s-Triazinderivate sind zum Beispiel gemäß FR 1.592 489 erhältlich.

In einer bevorzugten Ausführungsform läßt man die Reaktion in Gegenwart eines an sich bekannten Phasentransferkatalysators ablaufen, der dem folgenden Formelschema entspricht:

$[R_nNH_{4-n}]^+X^-$, worin n = 1, 2, 3 und 4
$[R_pPH_{4-p}]^+X^-$, worin p = 3 von 4
$[R'C_5H_4NR'']^+X^-$, worin $C_5H_4N$ ein Pyridinring, und
$[R_1R_2R_3S]^+X^-$, worin bedeuten:

X = Halogen oder Hydroxid oder Hydrogensulfat ($HSO_4^-$)
R = eine Alkylgruppe mit 1 - 18 C-Atomen
eine Alkenylgruppe mit 1 - 18 C-Atomen
eine Phenylgruppe, eine Benzylgruppe,
wobei die Verbindungen 1 und 2 nicht mehr als eine dieser Phenyl- und Benzylgruppen enthalten.
R' = Wasserstoff, eine Alkyl- oder Alkylengruppe mit 1 - 4 C-Atomen
R'' = eine Alkyl- oder Alkenylgruppe mit 1-18 C-Atomen
$R_1$, $R_2$ und $R_3$ = eine Alkyl- oder Alkenylgruppe mit 1 - 18 C-Atomen oder eine Phenylgruppe.

Diese Katalysatoren werden in einer Menge von 0,1 - 5 Gew.-%, bezogen auf die s-Triazinverbindung der allgemeinen Formel II, eingesetzt.

Die erfindungsgemäßen Verbindungen gemäß Formel (I) zeichnen sich bei ihrer Verwendung als Vulkanisationsbeschleuniger gegenüber dem nach dem Stand der Technik bekannten Bis(2-ethylamino-4-diethylamino-s-triazin-6-yl)tetrasulfan - je nach Kautschuktyp - durch eine höhere Mooney- Scorchzeit, eine deutlich längere Anvulkanisationszeit, eine erhöhte Vernetzungsgeschwindigkeit und besseres Reversionsverhalten aus.

Ein weiterer heute nicht mehr zu vernachlässigender Nachteil mancher konventioneller Beschleuniger (z. B. mancher Sulfenamide, Thiurame) liegt darin, daß während des Vulkanisationsprozesses Amine freigesetzt werden können, die -soweit sie nitrosierbar sind- zur Bildung von Nitrosaminen im Vulkanisat führen, die -soweit sie toxisch sind- auf Dauer eine Beschränkung der Einsatzmöglichkeiten dieser Beschleuniger erwarten lassen.

Die Verwendung der erfindungsgemäß beanspruchten Verbindungen gemäß Formel (I) umfaßt die nach dem Stand der Technik bekannten Kautschukmischungen auf der Basis von Naturkautschuk (NR), Isoprenkautschuk (IR), Butadienkautschuk (BR), Styrol-Butadienkautschuk (SBR), Isobutylen-Isoprenkautschuk (IIR), Ethylen-Propylen-Terpolymer (EPDM), Nitrilkautschuk (NBR), halogenhaltige Kautschuke und auch epoxidierte Naturkautschuke (ENR) sowie deren Verschnitte. Besondere Bedeutung hat die Verwendung der erfindungsgemäß beanspruchten Verbindungen im Falle der reversionsanfälligen Kautschuktypen wie zum Beispiel Naturkautschuk, Isopren- und Butadien-Kautschuke sowie deren Verschnitte untereinander oder mit anderen Kautschuken.

Bei der beschleunigten Schwefelvulkanisation setzt man die erfindungsgemäß beanspruchten Bis(2,4-organylthio-s-triazin-6-yl)polysulfane als Beschleuniger in Mengen von 0,01 bis 10 Teilen, vorzugsweise 0,1 bis 5 Teile, bezogen auf 100 Teile Kautschuk, ein bei Schwefeldosierungen von 0,1 bis 10 Teilen. Bevorzugt wird ein molares Verhältnis der erfindungsgemäßen Beschleuniger zu Schwefel ($S_8$) von 1 : 0,5 bis 1,5. Zur Erzielung einer gewissen Variationsbreite der Vulkanisationskinetik kann es sich dabei als zweckmäßig erweisen 2 oder mehrere Bis(2,4-organylthio-s-triazin-6-yl)polysulfane im Gemisch einzusetzen, wobei zur Einhaltung der obengenannten Anwendungsmengen, insbesondere des bevorzugten Beschleuniger/-Schwefelverhältnisses, die Substitution auf molarer Basis vorzunehmen ist.

Ebenfalls aus kinetischen Gründen kann es sich als zweckmäßig erweisen, Bis(2,4-organylthio-s-triazin-6-yl)polysulfan im Gemisch mit konventionellen Beschleunigern,wie z. B. Sulfenamiden und Thiuramen, zu verwenden. Diese Maßnahmen gehen gelegentlich zu Lasten der Reversionsbeständigkeit im Vergleich zu den reinen Bis(2,4-organylthio-s-triazin-6-yl)polysulfan-Vulkanisaten.

Indessen wirkt sie sich hinsichtlich des Reversionsverhaltens umgekehrt positivaus, wenn man konventionelle Beschleuniger partiell durch Bis(2,4-organylthio-s-triazin-6-yl)polysulfane ersetzt.

Eine weitere wesentliche Beeinflussung der Inkubationszeit bei Verwendung der erfindungsgemäßen Verbindungen in Kautschukmischungen kann durch Kombination mit handelsüblichen Vulkanisationsverzögerern, wie z. B. Santoguard(R) PVI (N-(Cyclohexylthio)-phthalimid),Vulkalent (R) E (N-Phenyl-N-(trichlormethylsulfenyl)benzolsulfinamid) sowie Substanzen, wie sie in den südafrikanischen Patenten 87/1767 und 87/1768 und im US-Patent 3,546,185 beschrieben worden sind, erreicht werden. Durch deren steigenden Zusatz erfolgt ein linearer Anstieg der Inkubationszeit Bis(2,4-organylthio-s-triazin-6-yl)polysulfan-enthaltender Mischungen.

Die Verzögerer, insbesondere die oben genannten, und die erfindungsgemäßen Verbindungen gemäß der allgemeinen Formel (I) werden im Molverhältnis 0,5 bis 1,5 : 1, insbesondere 0,8 bis 1,2 : 1 bei einer Schwefeldosierung von 0,1 bis 10 Gew.-Teilen, vorzugsweise 0,5 bis 8 Gew.-Teilen, bezogen auf 100 Teile Kautschuk, eingesetzt.

Die Verwendung der erfindungsgemäßen Verbindung gemäß den Ansprüchen 1 bis 3 erfolgt in Kautschukmischungen, die weitere übliche Komponenten enthalten können, wie zum Beispiel:

- übliche Verstärkungssysteme, d. h. Furnace -Ruße, Channel-Ruße, Flammruße, Thermalruße, Acetylruße, Lichtbogenruße, CK-Ruße usw., sowie synthetische Füllstoffe wie Kieselsäuren, Silikate, Aluminiumoxidhydrate, Calciumcarbonate und natürliche Füllstoffe wie Clays, Kieselkreiden, Kreiden, Talke usw., sowie silanmodifizierte Füllstoffe und deren Verschnitte in Mengen von 5 bis 300 Teilen, bevorzugt sind insbesondere Kieselsäuren und Silikate, auch als alleinige Füllstoffe in Mengen von 5 bis 150 Teilen oder in Gemischen mit Ruß mit 10 bis 100 Teilen, jeweils bezogen auf 100 Teile Kautschuk
- ZnO und Stearinsäure als Promotoren der Vulkanisation in Mengen von 0,5 bis 10 Teilen, je 100 Teile Kautschuk,
- üblicheweise verwendete Alterungs-, Ozon-, Ermüdungsschutzmittel wie zum Beispiel IPPD, TMQ sowie auch Wachse als Lichtschutzmittel und deren Verschnitte,
- beliebige Weichmacher wie zum Beispiel aromatische, naphthenische, paraffinische, synthetische Weichmacher und deren Verschnitte,
- gegebenenfalls Silane wie zum Beispiel Bis(3-triethoxysilylpropyl)tetrasulfan, 3-Chlorpropyltriethoxysilan, 3-Mercaptopropyltrimethoxysilan,

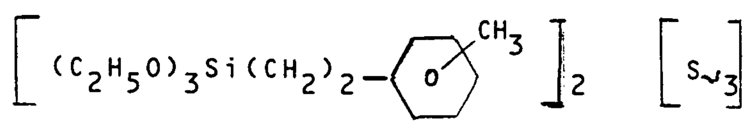

  und deren Verschnitte in einer Menge von 0,1 bis 20 Teilen, bevorzugt 1 bis 10 Teile, je 100 Teile Füllstoff, gegebenenfalls Schwefel in einer Menge von 0,5 bis 4 Teilen, je 100 Teile Kautschuk,
- gegebenenfalls weitere Silane wie Chlorpropyltrialkoxysilane, Vinyltrialkoxysilane und Aminoalkyltrialkoxysilane wie deren Verschnitte in einer Menge von 0,1 bis 15 Teilen, bevorzugt 1 bis 10 Teile, je 100 Teile silanolgruppentragender Füllstoffe wie Kieseläsuren, Silikate, Clays usw.
- gegebenfalls Farbstoffe und Verarbeitungshilfsmittel in der üblichen Dosierung

Der Anwendungsbereich der Bis(2,4-organylthio-s-triazin-6-yl)polysulfane erstreckt sich auf Kautschukmischungen gemäß den Ansprüchen 4 bis 7, wie sie üblicherweise im Reifenbau verwendet werden und auf technische Artikel, wie zum Beispiel Mischungen für Fördergurte, Keilriemen, Formartikel, Schläuche mit und ohne Einlagen, Walzengummierungen, Auskleidungen, Spritzprofile, Freihandartikel, Folien, Schuhsolen und Oberteile, Kabel, Vollgummireifen und deren Vulkanisate.

Die Herstellung der vulkanisierbaren Mischungen erfolt nach allgemein bekannten Verfahren.
Man benutzt beispielsweise einen Innenmischer mit einer Durchflußtemperatur von ca. 60 °.

In diesem wird die schwefel- und beschleunigerfreie Kautschukmischung vorgemischt und anschließend der Beschleuniger und der gegebenenfalls ebenfalls vorgesehene Schwefel und/oder ein Organosilan als Haftvermittler in einem zweiten Schritt eingemischt.

Beispiele

Die Darstellung von Bis(2,4-methylthio-s-triazin-6-yl)polysulfan erfolgt nach folgender Gleichung:

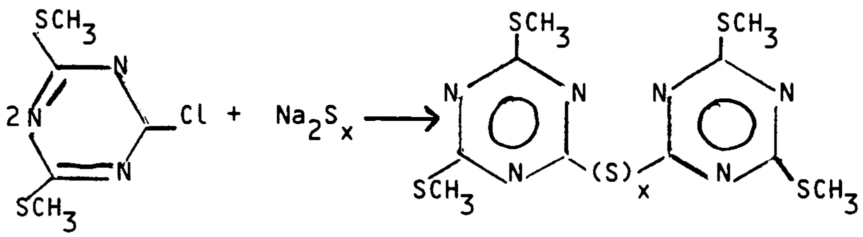

Beispiel 1

250 ml-Mehrhalskolben mit Rührer, Kühler, Thermometer

50 mmol (=10,39 g) 2,4-Dimethylmercapto-6-chlor-s-triazin (BMT)
30 mmol ( 5,23 g) Dinatriumtetrasulfid
100 ml Tetrahydrofuran (THF)

Das THF wird bei Raumteperatur vorgelegt. Unter Rühren trägt man das gemörserte BMT ein. Nach vollständiger Auflösung gibt man unter kräftigem Rühren (800 Upm) zügig das feingemörserte $Na_2S_x$ ($\bar{x}$ ~ 4) zu. Nach bis zu 5 Minuten entsteht ein weißer Niederschlag, die Temperatur steigt bis auf 30 °C an.

Die Reaktionsgeschwindigkeit kann durch Zugabe von 0,3 Gew.-% eines Phasentransferkatalysators z. B. Benzyltriehtylammoniumchlorid (TEBA) - erhöht werden.

Nach 60 Minuten saugt man den Niederschlag ab, wäscht mit Wasser choridfrei und trockent über $P_2O_5$.

Ausbeute: 90 % Polysulfan ($\bar{x}$ ~ 4)
Fp: 169 - 178 °C

Beispiel 2

250 ml Mehrhalskolben, Rührer, Kühler, Thermometer, Tropfrichter (50 ml)

Die eingesetzten Mengen sind dieselben wie in Beispiel 1. Das THF wird bei Raumtemperatur vorgelegt. Unter Rühren trägt man das gemörserte BMT ein. Nach vollständiger Auflösung tropft man eine Lösung aus $Na_2S_x$ ($\bar{x}$ ~ 4) und 25 ml Wasser zu (Rührgeschwindigkeit ca. 800 Upm).

Nach wenigen Minuten entsteht ein weißer Niederschlag, die Temperatur steigt bis auf ca. 30 °C an.

Ausbeute: 89 % Polysulfan ($\bar{x}$ ~ 4)
Fp: 160 - 180 °C

Die Identifizierung der hergestellten Verbindung erfolgt durch

1) Elementaranalyse: $C_{10}H_{12}N_6S_8$

| | C | H | N | S | Cl |
|---|---|---|---|---|---|
| ber.: | 27,26 | 2,74 | 19,07 | 50,93 | 0 |
| gef.: | 27m13 | 2,75 | 18,80 | 51,09 | 0,27 |

2) IR-Spektren
IR ($cm^{-1}$): 3435, 2929, 1499, 1475, 1426, 1323, 1250, 1160, 844, 783

3) NMR
H-NMR ($CDCl_3$, 250 MHz): s. 2,58 ppm
$^{13}C$-NMR ($CDCl_3$), 250 MHz): ppm: 13,5 (-S-$\underline{C}H_3$), 177,187

Prüfungsnormen für die Anwendung:

Die physikalischen Prüfungen wurden bei Raumtemperatur nach folgenden Normvorschriften ausgeführt:

| | | gemessen in: |
|---|---|---|
| Zugfestigkeit, Bruchdehnung und Spannungswert an 6 mm starken Ringen | DIN 53 504 | MPa |
| Reversion | DE-PS 2848559 | % |
| Scorchzeit | ASTM D 2084 | min. |
| Weiterreißwiderstand | DIN 53 507 | N/mm |
| Mooney-Prüfung | DIN 53 523/524 | min. |

In den Anwendungsbeispielen werden folgende Namen und Abkürzungen benutzt, deren Bedeutung um folgende angegeben wird:

| | |
|---|---|
| Buna 1500: | Styrol-Butadienkautschuk der Firma Hüls |
| RSS: | Ribbed Smoked Sheet (Naturkautschuk) |
| CORAX (R) N 220: | Ruß, Oberfläche (BET) 120 $m^2/g$ (DEGUSSA) |
| Naftolen(R) ZD: | Weichmacher aus Kohlenwasserstoffen |
| Vulkanox(R) 4010 NA: | N-Isoproypyl-N'-phenyl-p-phenylen-diamin |
| Vulkanox(R) HS: | Poly-2,2,4-trimethyl-1,2-dihydro-chinolin |
| Protektor(R)G35: | Ozonschutzwachs |
| V 480: | Bis(2-ethylamino-4-diethylamino-s-triazin-6yl)tetrasulfan |
| V 675: | Bis(2,4-methylthio-s-triazin-6-yl)polysulfan($\bar{x}$ ~ 4) |

Beispiel 3

Vergleich des gummitechnischen Wertebildes von Bis(2,4-methylthio-s-triazin-6-yl)polysulfan (V 675) mit Bis(2-ethylamino-4-diethylamino-s-triazin-6-yl) tetrasulfan (V 480) in Naturkautschuk

| | | |
|---|---|---|
| RSS 1 | 100 | 100 |
| CORAX N 220 | 50 | 50 |
| ZnO RS | 5 | 5 |
| Stearinsäure | 2 | 2 |
| Naftolen ZD | 3 | 3 |
| Vulkanox 4010NA | 2,5 | 2,5 |
| Volkanox HS | 1,5 | 1,5 |
| Protektor G35 | 1 | 1 |
| V 480 | 3,5 | - |
| V 675 | - | 3,1 |
| Schwefel | 0,3 | 1 |

Vulkanisationstemperatur: 160 °C
Vulkanisatdaten bei 160 °C, $T_{95}$%

| | | |
|---|---|---|
| Vulkanisationszeit (min) | 15 | 15 |
| $t_{10}$% (min) | 3,0 | 4,2 |
| $t_{80}$%- $t_{20}$% (min) | 3,8 | 2,4 |
| $t_{90}$%- $t_{10}$% (min) | 12,7 | 5,2 |
| MS $t_5$ (130 °C) (min) | 3,6 | 9,7 |
| MS $t_{35}$ (130 °C) (min) | 6,2 | 13,1 |
| Zugfestigkeit (MPa) | 21,2 | 21,9 |
| Modul 300 % (MPa) | 10,1 | 10,4 |
| Weiterreißwiderstand (N/mm) | 21 | 30 |

Beispiel 4

Reversionsstabilität von mit Bis (2,4-methylthio-s-triazin-6-yl)polysulfan (V 675) beschleunigten N 220-gefülltem SBR im Vergleich zu Bis(2-ethylamino-4-diethylamino-s-triazin-6-yl)tetrasulfa (V 480)

| | | |
|---|---|---|
| Buna | 100 | 100 |
| CORAX N 220 | 50 | 50 |
| ZnO RS | 5 | 5 |
| Stearinsäure | 2 | 2 |
| Naftolen ZD | 3 | 3 |
| Vulkanox 4010NA | 2,5 | 2,5 |
| Vulknox HS | 1,5 | 1,5 |
| Protektor G35 | 1 | 1 |
| V 480 | 1 | - |
| V 675 | - | 2 |
| Schwefel | 1,8 | 2,5 |

Vulkanisationstemperatur: 170 °C

Reversion:

$$\frac{Dmax-D(max + 60')}{Dmax - Dmin} \ (\%) \quad 8,3 \qquad 3,9$$

## Patentansprüche

**1.** Bis(2,4-organylthio-s-triazin-6-yl)polysulfane der allgemeinen Formel (I)

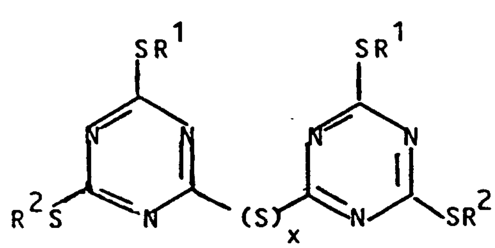

in der bedeuten:
$R^1$, $R^2$: gleich oder verschieden, H, Alkyl mit 1 - 4 C-Atomen, verzweigt oder unverzweigt, Allyl, $C_3$-$C_8$ Cycloalkyl, 2-Hydroxyethyl, 3-Hydroxypropyl, 2-Hydroxypropyl, Phenyl, $(S)_x$ eine Polysulfankette mit 2-10 S-Atomen ($2 \leq x \leq 10$), wobei die einzelnen Polysulfane in solchen Konzentrationen vorliegen, daß das statistische Mittel $\bar{x}$ ganze oder gebrochene Zahlenwerte von 2 bis 5 annimmt, mit Ausnahme des Bis (2,4-dimethylthio-s-triazin-6-yl)disulfids und des Bis (2,4-dithiol-s-triazin-6-yl)disulfids.

**2.** Verfahren zur Herstellung von Verbindungen gemäß Anspruch 1, dadurch gekennzeichnet, daß man eine s-Triazinverbindung der allgemeinen Formel (II)

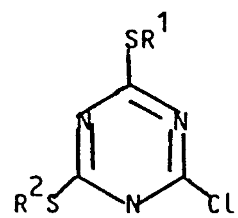

in der $R^1$ und $R^2$ die oben angegebenen Bedeutungen besitzen, in einem organischen Lösungsmittel oder deren Gemischen mit Wasser mit einer Verbindung der allgemeinen Formel (III)

$Me_2S_x$ ,

in der Me für das Ammonium- oder ein Alkalikation steht, und $S_x$ der obengenannten Bedeutung entspricht, in einem Molverhältnis von 2 : 1 bis 2 : 1,2 bei einer Temperatur von 0 °C bis zum Siedepunkt des organischen Lösungsmittels umsetzt und das Produkt anschließend abtrennt.

**3.** Verfahren gemäß Anspruch 2, dadurch gekennzeichnet, daß man die Reaktion in Gegenwart eines Phasentransferkatalysators ablaufen läßt, der dem folgenden Formelschema entspricht:

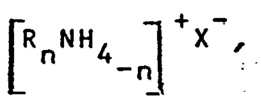

worin n = 1,2,3 und 4

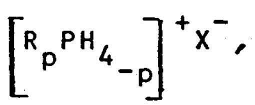

worin p = 3 von 4
$[R'C_5H_4NR'']^+X^-$, worin $C_5H_4N$ ein Pyridinring, und
$[R_1R_2R_3S]^+X^-$,
worin bedeuten:

| | |
|---|---|
| X = | Halogen oder Hydroxid, Hydrogensulfat ($HSO_4^-$) |
| R = | eine Alkylgruppe mit 1-18 C-Atomen<br>eine Alkenylgruppe mit 1-18 C-Atomen<br>eine Phenylgruppe, eine Benzylgruppe<br>wobei die Verbindungen 1 und 2 nicht mehr als eine dieser Phenyl- und Benzylgruppen enthalten. |
| R′ = | Wasserstoff, eine Alkyl- oder Alkylengruppe mit 1-4 C-Atomen |
| R″ = | eine Alkyl- oder Alkenylgruppe mit 1-18 C-Atomen |
| $R_1$, $R_2$ und $R_3$ = | eine Alkyl- oder Alkenylgruppe mit 1-18 C-Atomen oder eine Phenylgruppe |

**4.** Vulkanisierbare, Füllstoffe, Schwefel und weitere übliche Bestandteile enthaltende Mischungen auf der Basis eines oder mehrerer natürlicher und/oder synthetischer Kautschuk(e) (s),
dadurch gekennzeichnet, daß sie 0,01 bis 10 Teile, bevorzugt 0,1 bis 5 Teile, mindestens einer der Verbindungen gemäß Formel (I), gegebenenfalls zusammen mit konventionellen Bescheunigern enthalten, bei Schwefeldosierungen von 0,1 bis 10 Teilen, jeweils bezogen auf 100 Teile Kautschuk.

**5.** Vulkanisierbare Mischungen gemäß Anspruch 4,
dadurch gekennzeichnet, daß sie einen Verzögerer und mindestens eine der Verbindungen gemäß

Formel (I) im Molverhältnis 0,5 bis 1,5 : 1 enthalten.

**6.** Vulkanisierbare Mischungen gemäß den Ansprüchen 4 und 5,
dadurch gekennzeichnet, daß sie als Füllstoff nur Kieselsäure enthalten.

**7.** Vulkanisierbare Mischungen gemäß den Ansprüchen 4 und 5,
dadurch gekennzeichnet, daß sie als Füllstoff neben Ruß 10 bis 100 Teile Kieselsäure, bezogen auf 100 Teile Kautschuk, enthalten.

## Claims

**1.** Bis(2,4-organylthio-s-triazine-6-yl)polysulphanes of the general formula (I)

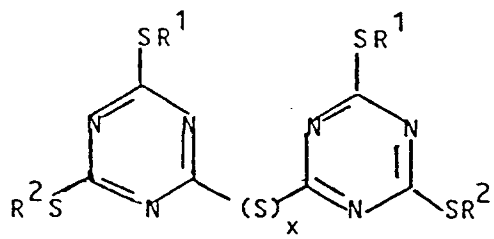

wherein
$R^1$, $R^2$ signify identical or different, H, alkyl having 1 to 4 C atoms, branched or unbranched, allyl, $C_3$-$C_8$ cycloalkyl, 2-hydroxyethyl, 3-hydroxypropyl, 2-hydroxypropyl, phenyl, $(S)_x$ signifies a polysulphane chain having 2 to 10 S atoms ($2 \leqq x \leqq 10$), wherein the individual polysulphanes are present in concentrations such that the statistical average $\bar{x}$ assumes integral or fractional numerical values of from 2 to 5, with the exception of bis(2,4-dimethylthio-s-triazine-6-yl)disulphide and of bis(2,4-dithio-s-triazine-6-yl) disulphide.

**2.** Method for preparing compounds according to claim 1, characterised in that an s-triazine compound of the general formula (II)

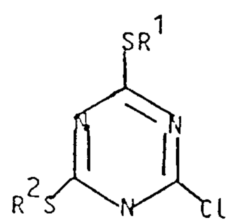

wherein $R^1$ and $R^2$ have the meanings given above, in an organic solvent or mixtures thereof with water, is reacted with a compound of the general formula (III)

$Me_2S_x$,

wherein Me represents the ammonium ion or an alkali cation and $S_x$ corresponds to the meaning given above, in a molar ratio of from 2 : 1 to 2 : 1.2 at a temperature of from 0 °C to the boiling point of the organic solvent and the product subsequently separated.

**3.** Method according to claim 2, characterised in that the reaction is caused to proceed in the presence of a phase-transfer catalyst corresponding to the following formulae:
$[R_nNH_{4-n}]^+ X^-$, wherein n = 1,2,3 and 4
$[R_pPH_{4-p}]^+ X^-$, wherein p = 3 out of 4
$[R'C_5H_4NR'']^+ X^-$, wherein $C_5H_4N$ is a pyridine ring,
and $[R_1R_2R_3S]^+ X^-$,

wherein

| | |
|---|---|
| X | signifies halogen or hydroxide, hydrogen sulphate ($HSO_4^-$) |
| R | signifies: |
| | an alkyl group having 1 to 18 C atoms |
| | an alkylene group having 1 to 18 C atoms |
| | a phenyl group, a benzyl group, wherein the compounds 1 and 2 contain no more than one of the said phenyl and benzyl groups |
| R' | signifies hydrogen, an alkyl or alkylene group having 1 to 4 C atoms |
| R'' | signifies an alkyl or alkylene group having 1 to 18 C atoms |
| $R_1$, $R_2$ and $R_3$ | signify an alkyl or alkylene group having 1 to 18 C atoms or a phenyl group. |

**4.** Vulcanisable mixtures based on one or several natural and/or synthetic rubber(s) (s) and containing fillers, sulphur and other conventional components, characterised in that they contain from 0.1 to 10 parts, preferably 0.1 to 5 parts, of at least one of the compounds according to formula (I), optionally together with conventional accelerators, at sulphur dosages of from 0.1 to 10 parts, in each case referred to 100 parts of rubber.

**5.** Vulcanisable mixtures according to claim 4, characterised in that they contain a retarder and at least one of the compounds according to formula (I) in the molar ratio of from 0.5 to 1.5 : 1.

**6.** Vulcanisable mixtures according to claims 4 and 5, characterised in that they contain only silicic acid as a filler.

**7.** Vulcanisable mixtures according to claims 4 and 5, characterised in that they contain as a filler, in addition to carbon black, 10 to 100 parts of silicic acid, referred to 100 parts of rubber.

**Revendications**

**1.** Bis(2,4-organylthio-s-triazine-6-yl)polysulfanes de formule générale (I)

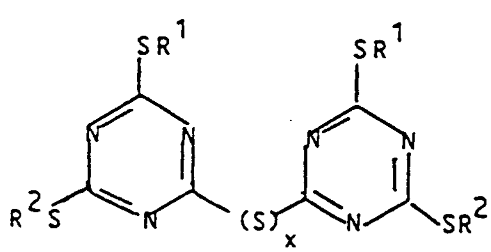

dans laquelle :

$R^1$, $R^2$ sont égaux ou différents et représentent, H, un alkyle avec 1-4 atomes de carbone, ramifiés ou non ramifiés, un alkyle, un cycloalkyle en $C_3$-$C_8$, en particulier du cyclohéxyle, un phényle ou aussi un 2-hydrohéxyle, un 3-hydroxypropyle, un 2-hydroxypropyle, $(S)_x$ une chaîne de polysulfane avec 2-10 atomes de C ($2 \leqq x \leqq 10$), les polysulfanes individuels étant présents dans des concentrations telles que la moyenne statistique $\bar{x}$ prend des valeurs numériques entières ou fractionnaires de 2 à 5, 2 à l'exception du bis(2,4-diméthylthio-s-triazine-6-yl)disulfure et du bis (2,4-dithiol-s-triazine-6-yl)disulfure

**2.** Procédé de fabrication de composés selon la revendication 1, caractérisé en ce qu'on transforme un composé de s-triazine de formule générale (II)

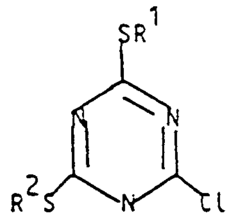

dans laquelle $R^1$ et $R^2$ ont les significations indiquées plus haut, en un solvant organique ou leurs mélanges avec de l'eau avec un composé de formule générale (III).

$Me_2S_x$,

dans laquelle Me représente l'ammonium ou un cation alcalin, et $S_x$ a la signification indiquées ci-dessus, dans un rapport molaire de 2:1 à 2:1,2 à une température de 0 ° C jusqu'au point d'ébullition du solvant organique et ensuite on sépare le produit.

**3.** Procédé selon la revendication 2, caractérisé en ce qu'on laisse se dérouler la réaction en présence d'un catalyseur de transfert de phase, qui correspond au schéma formulaire suivant :
$[R_nNH_{4-n}]^+x^-$, dans laquelle n = 1, 2, 3 et 4
$[R_pPH_{4-p}]^+x^-$, dans laquelle p = de 3 à 4
$[R'C_5H_4NR'']^+x^-$, dans laquelle $C_5H_4N$ représente un noyau pyridine et
$[R_1R_2R_3S]^+x^-$ dans laquelle

| | |
|---|---|
| x = | un halogène ou un hydroxyde ou du sulfate d'hydrogène ($HSO_4^-$) |
| R = | un groupe alkyle avec de 1 à 18 atomes de C<br>un groupe alcényle avec de 1 à 18 atomes de C<br>un groupe phényle, un groupe benzyle, les composés 1 et 2 ne contenant pas plus qu'un de ces groupes phényle et benzyle. |
| R' = | de l'hydrogène, un groupe alkyle ou alkylène avec de 1 à 4 atomes de C. |
| R'' = | un groupe alkyle ou alcényle avec de 1 à 18 atomes de C. |
| $R_1$, $R_2$ et $R_3$ = | un groupe alkyle ou alcényle avec de 1 à 18 atomes de C ou un groupe phényle. |

**4.** Mélanges vulcanisables contenant des charges, du soufre et d'autres composants usuels, à base d'un ou de plusieurs caoutchouc(s) (s) naturels(s) et/ou synthétique(s), caractérisés en ce qu'ils contiennent de 0,01 à 10 parties, de préférence de 0,1 à 5 parties, au moins d'un des composés selon la formule (I), éventuellement ensemble avec des accélérateurs conventionnels, avec des dosages de soufre de 0,1 10 parties par rapport respectivement à 100 parties de caoutchouc.

**5.** Mélanges vulcanisables selon la revendication 4, caractérisés en ce qu'ils contiennent un retardateur et au moins un des composés selon la formule (I) dans le rapport molaire de 0,5 à 1,5:1.

**6.** Mélanges vulcanisables selon les revendications 4 et 5, caractérisés en ce qu'ils contiennent comme charge seulement de l'acide silicique.

**7.** Mélanges vulcanisables selon les revendications 4 et 5, caractérisés en ce qu'ils contiennent comme charge outre du noir de carbone de 10 à 100 parties d'acide silicique, par rapport à 100 parties de caoutchouc.